# EUROPEAN PATENT APPLICATION

(11) **EP 2 074 932 A1**
(43) Date of publication of application: **01.07.2009**
(21) Application number: 08171312.5
(22) Date of filing: 11.12.2008
(51) Int. Cl.: A61B 1/01, A61B 1/273, A61B 1/313, A61B 17/34, A61M 25/01, A61M 39/22

(54) **Insertion assisting tool for an endoscope**

(30) Priority: 27.12.2007 JP 2007336136
(71) Applicant: Covidien AG, 8212 Neuhausen (CH)
(72) Inventor: Sutoh, Dai, Fukuroi-shi Shizuoka 437-0004 (JP)
(74) Representative: Körber, Martin Hans

(57) **Abstract**

An insertion assisting tool (20) when inserting an endoscope (30) into the gastric fistula catheter (10) that has been inserted in the fistula formed in the patient's body comprises a cylindrical body (21) that can be engaged with a gastric fistula catheter (10) wherein an endoscope can be inserted through a valve press member (22) and a sealing member (23). The valve press member (22) can be installed detachably by screwing the screw threads with the edge portion at an opposite side of the portion engaged with the gastric fistula catheter in the cylindrical body, and is formed in a cap form having a hole portion in the sealing portion. The sealing member (23) seals the space between the cylindrical body and the endoscope when it is pressed against the endoscope set at the space between the end portion of the cylindrical body and the valve press member.

## Description

### FIELD OF THE INVENTION

The present invention relates to an insertion assisting tool for an endoscope that may be used when inserting an endoscope into a gastric fistula catheter that has been inserted in a fistula opening formed in a patient's body.

### BACKGROUND OF THE INVENTION

Conventionally, an endoscope is inserted through a patient's nostril or mouth into the stomach in order to observe the gastric wall, but this method may hurt the patient. For this reason, recently, an endoscope is inserted into the gastric fistula catheter that has been inserted in the fistula formed in a patient's body in order to examine the inside of the stomach or to check the insertion position of the gastric fistula catheter. In this case, in order to check the status of the inside of the stomach and the insertion position of the gastric fistula catheter accurately, it is necessary to secure a space wherein the tip of the endoscope can move by increasing the spatial area inside of the stomach. For this purpose, air is blown into the interior cavity of the stomach. Further, in order to prevent leakage of air blown into the stomach out of the body, the space between the gastric fistula catheter and the endoscope must be sealed for air tightness and liquid tightness. For this purpose, an insertion assisting tool for an endoscope is used (see, Japanese Patent Application Publication No. 2000-126121, for example).

The existing valve device for a medical instrument (insertion assisting tool for an endoscope) comprises a two-state tube-form hard insertion tube and a cylindrical valve member made of an elastic material that is joined together detachably with the circumferential grooves formed in the inside periphery of the large-sized area of the insertion tube. A sheet valve portion that tightly contacts the outer surface of the treatment device (an endoscope) is provided in the valve member. When the treatment tool is inserted through the valve device, the space between the valve portion and the treatment tool can be sealed. However, since frictional resistance between the valve portion and the treatment device is small, the treatment device moves easily relative to the valve device, causing the operation to become unstable. Further, since the valve portion is formed on the internal circumferential surface of the valve member, such that frictional resistance with the treatment device becomes small, it becomes difficult to securely prevent leakage of air from the interior of the stomach to the outside of the body.

For this reason, a treatment device fixing mechanism enabling fixation of the treatment device was developed (see, Japanese Patent Application Publication No. 2007-075405, for example). This treatment device fixing mechanism comprises a treatment device insertion cap passing the treatment device (an endoscope) and a brake member which moves back and forth relative to the inside of the treatment device insertion cap by rotation of the operational unit. For this reason, the brake member can be pressed and fixed on the treatment device by the rotation of the operational unit in such a state that the treatment device is passed through the treatment device insertion cap. (see, Japanese Patent Application Publication No. 2000-126121 and Japanese Patent Application Publication No. 2007-075405, for example)

However, according to the aforementioned treatment device fixing mechanism, the problem is that a mechanism for fixing the treatment device and a mechanism for preventing air leakage are installed separately, making the structure more complex. Further, even with this treatment device fixing mechanism, a slit formed in the forceps plug constituting the sealed portion has the same constitution as the valve portion of said valve device for the medical instrument. Therefore, the drawback is that it is difficult to securely prevent air leakage from the interior of the stomach to the outside of the body.

### SUMMARY OF THE INVENTION

In one aspect of the present invention, an insertion assisting tool for an endoscope generally comprises of a tube-form portion having an internal cavity and an outer fixing portion forming an insertion hole passing through the cavity of the tube-form portion inside connected to a base end of the tube-form portion, wherein an insertion hole reaching the tube-form portion, that is used when inserting an endoscope into the gastric fistula catheter inserted into the fistula opening while the tube-form portion is located in the fistula opening formed in the space between the patient's skin surface and the interior surface of the gastric wall, comprises a cylindrical body that is engaged with the outer fixing portion for air tightness or liquid tightness wherein an endoscope can pass through, and a sealing member can be attached on the cylindrical body for sealing the space between the cylindrical body and the endoscope.

### BRIEF DESCRIPTION OF THE FIGURES

Fig. 1 is a gastric fistula catheter used in the embodiment of the present invention: (a) is a planar view, (b) is a front view, and (c) is a bottom view;

Fig. 2 is a front view showing an insertion assisting tool concerned in the first embodiment of the present invention;

Fig. 3 is a cross-sectional view of the insertion assisting tool shown in Fig. 2;

Fig. 4 is a front view showing a fiber scope;

Fig. 5 is a front view showing a protective cover;

Fig. 6 is a front view showing the status wherein the fiber scope having the protective cover is attached to the insertion assisting tool;

Fig. 7 is a partially notched cross-sectional view showing the status when positioning the fiber scope shown in Fig. 6 above the gastric fistula catheter inserted into patient's body;

Fig. 8 is a partially notched cross-sectional view showing the status when inserting the fiber scope shown in Fig. 6 into the gastric fistula catheter inserted into patient's body;

Fig. 9 is a partially notched cross-sectional view showing the status when imaging the inside of the stomach with the fiber scope;

Fig. 10 is a front view showing an insertion assisting tool concerned in the second embodiment of the present invention;

Fig. 11 is a front view showing the status when the insertion assisting tool shown in Fig. 10 is disassembled;

Fig. 12 is a cross-sectional view showing the status when the insertion assisting tool shown in Fig. 10 is disassembled;

Fig. 13 is a front view showing an insertion assisting tool concerned in the third embodiment of the present invention;

Fig. 14 is a front view showing the status when the insertion assisting tool shown in Fig. 13 is disassembled; and

Fig. 15 is a cross front view showing an insertion assisting tool concerned in the fourth embodiment of the present invention.

### DESCRIPTION OF FIGURE NOTATIONS

10: Gastric fistula catheter
11: External fixing unit
14: Insertion hole
14a: Valve body
20, 20a, 20b, 20c: Insertion assisting tools
21, 41: Cylindrical bodies
22, 42: Valve press members
22a: Hole portion
23, 43: Sealing member
24, 44: Branching tube
25: Insertion through-hole
26: Connection unit
30: Fiber scope
AW: Abdominal wall
S: Stomach
SW: Gastric wall

### DETAILED DESCRIPTION OF THE INVENTION

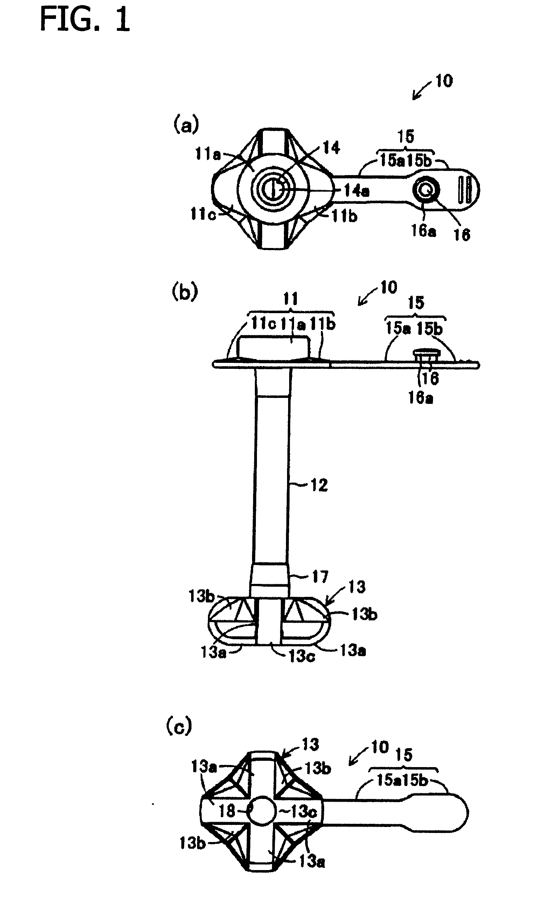

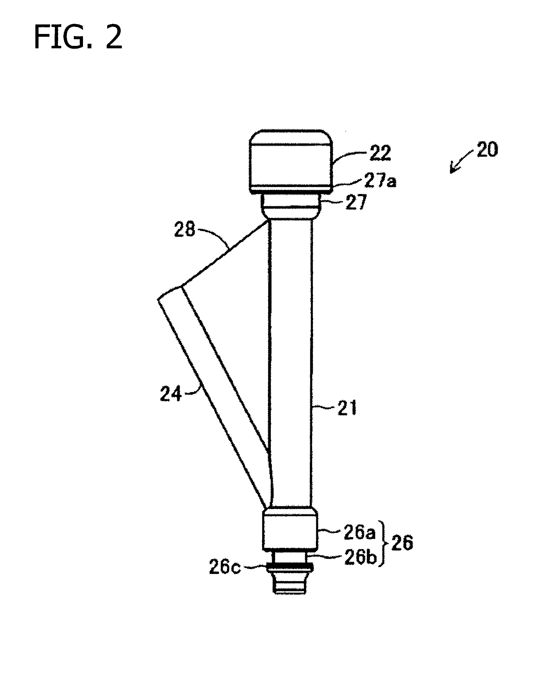

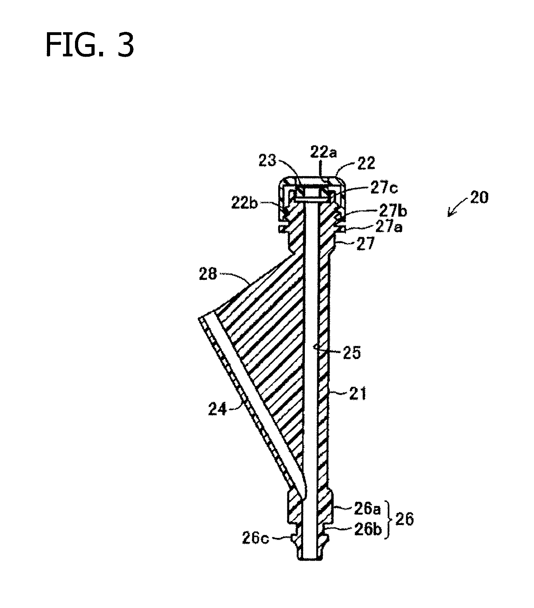

The first embodiment of the present invention will be explained below with reference to the drawings. Figure 1 shows a gastric fistula catheter 10 used in this embodiment. Figure 2 shows an insertion assisting tool 20 as an insertion assisting tool for an endoscope as concerned in the present embodiment that is installed in the gastric fistula catheter 10, while Figure 3 shows a longitudinal cross-sectional view of the insertion assisting tool 20 as shown in Figure 2. The gastric fistula catheter 10 comprises an outer fixing portion 11 made of a soft plastic material such as polyurethane or silicone, a tube-form portion 12 connected at the center of the lower face of the outer fixing portion 11, and a intra-gastric fixing portion 13 installed at the lower end of the tube-form portion 12. Hereinafter, the side of the outer fixing portion 11 is called an upper side and the side of the intra-gastric fixing portion 13 is called a lower side in the following description.

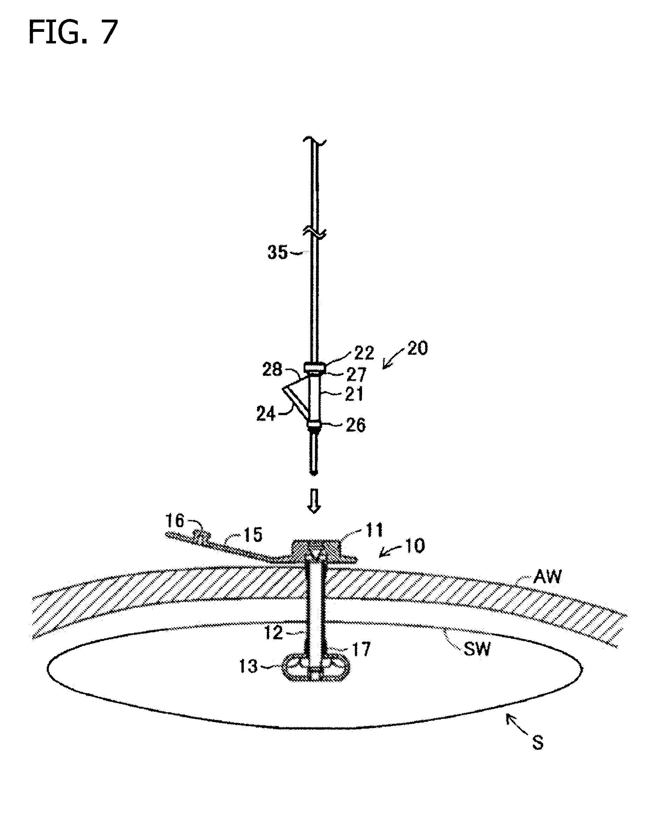

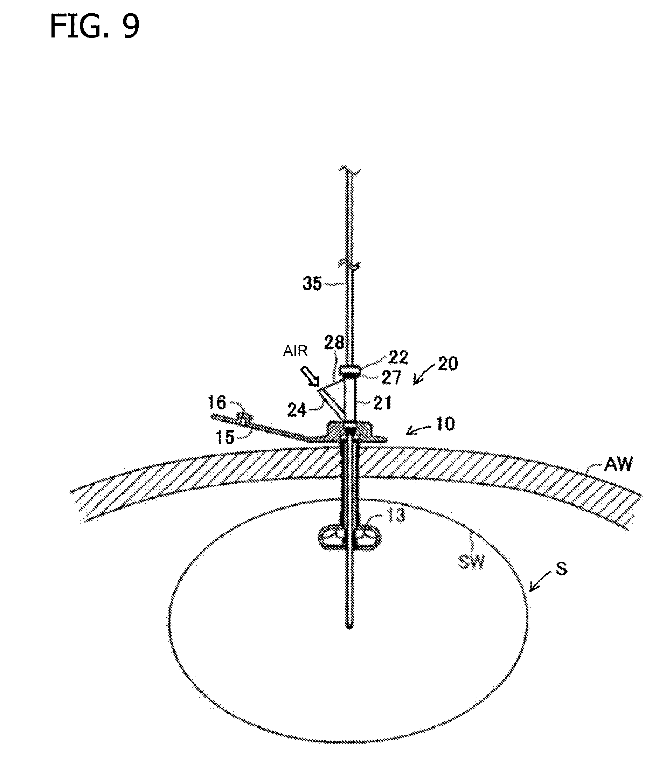

The outer fixing portion 11 comprises an insertion inlet portion 11a formed in a slightly thick ring form, and projected segments 11b and 11c that are roughly formed in a respective oval shape including the insertion inlet portion 11a from the plane view projected respectively at both sides and from both sides of the lower end of the insertion inlet portion 11a. These projected segments 11b and 11c have a function of preventing the gastric fistula catheter 10 from being pulled into the inside of the stomach S (See Fig. 7 or Fig. 9). A valve body 14a having a slit in the center is installed on the inner circumferential surface of the insertion hole 14 penetrating up and down formed in the center of the insertion inlet portion 11a. This valve body 14a has an elasticity such that when pressed, it is deformed to open the slit. Although it is not shown, an engagement groove is formed along the circumference on the upper side of the valve body 14a on the inner circumferential surface of the insertion hole 14. In addition, a cap portion 15 is connected at the tip of the projected segment 11b in order to close the insertion hole 14 of the insertion inlet portion 11a.

The cap portion 15 comprises a slender band-shaped connection portion 15a connected at the tip of the projected segment 11b and a broader width portion 15b which is formed at the tip of the band-shaped connection portion 15a and which is broader in width and shorter in length than the band-shaped connection portion 15a. In addition, a columnar plug portion 16 which is shorter in length in the axial direction is installed on the surface possibly facing the insertion inlet portion 11a in the broader-width portion 15b. The band-shaped connection portion 15a is pliable so as to bend to turn in an up- and-down direction using the connection portion with the projected segment 11b as the center, or to bend at a sharp angle. The plug portion 16 is made on the portion of the side of the band-shaped connection portion 15a in the broad-width portion 15b so as to face the insertion hole 14 when the broad-width portion 15b is positioned at about the insertion inlet portion of 11a by bending the band-shaped connection portion 15a.

The plug portion 16 is formed in a columnar shape to be joined together with the insertion hole 14 and a ring-form projected portion 16a is formed on the outer circumference at the tip portion along the circumference so as to be detachably engaged with the engagement groove portion formed on the inner circumferential surface of the insertion hole 14. Therefore, if the band-shaped connection portion 15a is upwardly bent inverted to press the plug portion 16 against the insertion hole 14, the engagement groove and the ring-form projected portion 16a can be engaged so that the insertion hole 14 of the insertion inlet portion 11a can be closed air tightly. Further, the engagement between the plug 16 and the insertion hole 14 can be released by pulling out the broad-width portion 15b so that the insertion hole 14 of the insertion inlet portion 11a can be opened.

The tube-form portion 12 is formed in a cylindrical shape wherein a supply path (not shown) as an internal cavity concerned in the present invention is formed for passing a liquid material such as nutrients, liquid foods and the like, and the upper end of the supply path is connected to the insertion hole 14 of the outer fixing portion 11. The intra-gastric fixing portion 13 is connected to the tube-form portion 12 via a joint portion 17 fixed at the lower end portion of the tube-form portion 12. This joint portion 17 is formed in a cylindrical form covering the outer circumferential face of the tube-form portion 12 and then integrated with the intra-gastric fixing portion 13.

The intra-gastric fixing portion 13 comprises four band-form curved portions 13a that are connected to the lower end opening portion of the joint portion 17 and then extended in the four directions, four connected film portions 13b that are installed on the upper portion of each band-form curved portions 13a and that form almost dome-shaped gastric wall contact portions with the four band-shaped curved portions 13a, and an aggregate portion 13c where tips of the respective band-shaped curved portions 13a are gathered. The four band-shaped curved portions 13a are configured such that they are separated from the lower end portion of the joint portion 17 into the four horizontal directions and extended downwards to be gathered to form an aggregated portion 13c constituting a band-shaped member curved nearly in a semi-circular form. The aggregate portion 13c connects among the respective band-shaped curved portions 13a by connecting the lower end portions of the respective band-shaped curved portions 13a, while its location is aligned below the central axis of the tube-form portion 12 by these band-shaped curved portions 13a.

The intra-gastric fixing portion 13 comprising band-shaped curved portions 13a, connected film portions 13b and an aggregate portion 13c is formed integral to the joint portion 17. Further, the band-shaped curved portions 13a and the connected film portions 13b are made of a soft elastic material having pliability so that a nearly spherical shape is generally maintained as an overall shape as shown in Fig. 1 due to elasticity, but when the aggregate portion 13c is pulled downwards, it is stretched to a straight slender state. Further, the space formed within the intra-gastric fixing portion 13 and the gaps formed among the lower portions of the respective band-shaped curved portions 13a serve as paths for passing fluids such as nutrients and liquid foods and the like carried from the supply path of the tube-form portion 12 into the stomach S. Furthermore, an insertion hole 18 is formed at the center of the aggregate portion 13c. The intra-gastric fixing portion 13 having this configuration has a function of preventing the gastric fistula catheter 10 aligned on the interior surface of the patient's gastric wall SW (See Fig. 7 or Fig. 9) from being removed from patient's body.

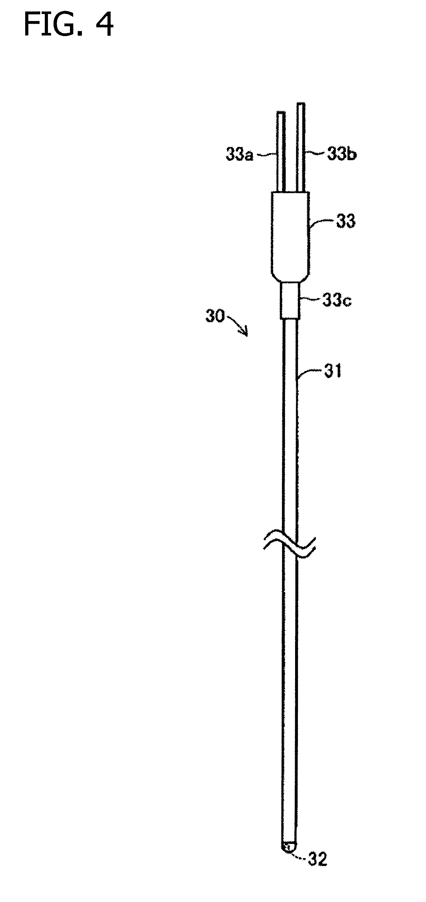

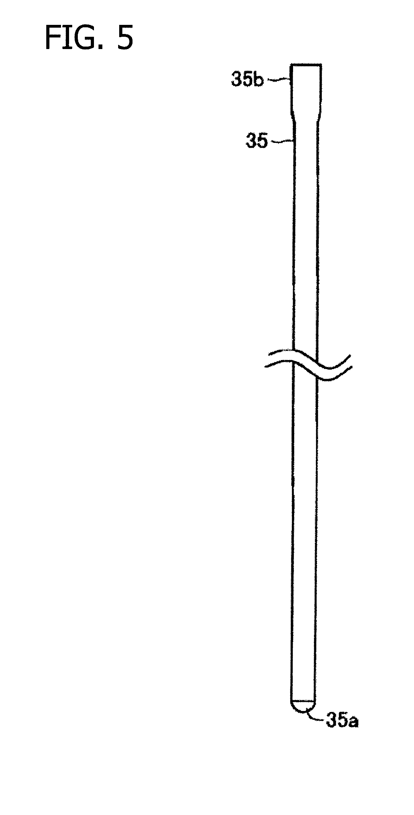

The insertion assisting tool 20, as shown in Fig. 2 and Fig. 3, comprises a cylindrical body 21, a valve press member 22, a sealing member 23 and a branching tube 24 branched from the cylindrical body 21. Further, the cylindrical body 21 is formed in a slender cylinder shape wherein an insertion hole 25 is formed such that a fiber scope 30 as will be described later (See Fig. 4) along with a protective cover 35 (See Fig. 5) can be inserted. A joint portion 26 is formed on its lower end portion and an insertion inlet portion 27 is formed at its upper end portion. Forming an engaged portion 26b on the body of the cylindrical joint portion 26a configures the joint portion 26. The body of the joint portion 26a is formed in a cylindrical form having a greater diameter than that of the insertion hole 14 of the gastric fistula catheter 10 and the engaged portion 26b is formed also in a cylindrical form having four different stages in sizes to be fitted within the insertion hole 14 of the gastric fistula catheter 10.

Further, the engaged portion 26b comprises an upper-most stage in a cylindrical shape, a second stage having a larger diameter than that of the upper-most stage that is shorter in length in the axial direction, a third stage having an inclined outer circumferential surface having a diameter of the upper end portion almost the same as that of the second stage and a diameter of the lower end portion smaller than that of the second stage, and a lower-most stage in a cylindrical shape having almost the same diameter as that of the lower end portion. In addition, the second stage of the engaged portion 26b constitutes a ring-shaped projection 26c to be joined detachably with an engagement groove portion formed in the insertion hole 14 of the gastric fistula catheter 10, and when the ring-shaped projection 26c is engaged with the engagement groove portion, the space between the engaged portion 26b and the circumferential surface of the insertion hole 14 becomes in an air-tight or liquid-tight state. Further, the lower end portion of the engaged portion 26b extends the slit of the valve body 14a formed in the insertion hole 14 so that the outer circumferential surface of the engaged portion 26b and the circumferential rim portion of the slit becomes a tightly sealed state.

The insertion inlet portion 27 is formed in a cylindrical shape having almost the same diameter as that of the body of the joint portion 26a, forming a flange-shaped stopper portion 27a nearly in the center in its axial direction. Further, a screw 27b is formed in the upper side portion of the stopper portion 27a in the outer circumferential surface of the insertion inlet portion 27. In addition, a storage concave portion 27c is formed for installing a sealing member 23 on the upper end internal circumferential surface of the insertion inlet portion 27. The branched tube 24 is formed in a cylindrical shape extending upwards diagonally in an inclined state with approximately an angle of 30 degrees relative to the cylindrical body 21 from the upper portion of the joint portion 26 in the cylindrical body 21 and has a smaller diameter than the cylindrical body 21.

An air supply apparatus (now shown) is connected to the tip portion of the branched tube 24 and the air supplied from the air supply apparatus is carried into the interior of the cylindrical body 21 through the interior of the branched tube 24. Further, an air path (not shown) is formed for passing air from the interior of the cylindrical body 21 to the interior lower end of the joint portion 26 such that air carried to the interior lower end of the cylindrical body 21 is discharged from the lower end of the joint portion 26 to the outside. In addition, a nearly triangular shaped thin plate reinforcement grip portion 28 is formed in order to reinforce the space between the cylindrical body 21 and the branched tube 24 as well as to make holding the insertion assisting tool 20 with a hand much easier.

The valve press member 22 is constructed in a cap form body in the sealing portion wherein a hole portion 22a having a slightly larger diameter than that of the insertion through-hole 25 of the cylindrical body 21 is formed and a screw thread 22b that can be screwed with the screw thread 27b of the cylindrical body 21 is formed on its interior circumferential surface. The screw thread 22b can be screwed in with the screw thread 27b until the lower end opening rim portion of the valve press member 22 is brought in contact with the stopper portion 27a of the insertion inlet portion 27. Further, the sealing member 23 is made of a deformable ring-shaped elastomer such as natural rubber, synthetic rubber, silicone and the like. The inner diameter of the sealing member 23 is almost equal to the inner diameter of the insertion through-hole 25 of the cylindrical body 21, while the outer diameter of the sealing member 23 is almost equal to the inner diameter of the hole portion 22a of the valve press member 22.

The insertion assisting tool 20 is assembled by setting the sealing member 23 within the storage concave portion 27c formed on the supper end surface of the cylindrical body 21 and subsequently by screwing the screw thread 22b of the valve press member 22 with the screw thread 27b of the cylindrical body 21. In this case, deformation of the sealing member 23 can be greater or smaller by adjusting the status of screwing between the screw thread 27b and the screw thread 22b. Accordingly, if the status of screwing between the screw thread 27b and the screw thread 22b is loosened (to shorten the length of the screw thread part), the sealing member 23 is slightly pressed such that the pressed part is projected slightly to the side of the interior circumference. In contrast, if the status of screwing between the screw thread 27 b and the screw thread 22b is tightened (to extend the length of the screw thread part), the sealing member 23 is greatly pressed such that the pressed part is largely projected to the side of the interior circumference.

The insertion assisting tool 20 thus constructed is incorporated in the gastric fistula catheter 10, and the fiber scope 30 shown in Fig. 4 along with the protective cover 35 is inserted into the interior of the insertion assisting tool 20 in such a state and into the gastric fistula catheter 10. The fiber scope 30 comprises a fiber scope shaft 31, lens 32 attached at the tip of the fiber scope shaft 31, and a joint portion 33 attached at the rear end of the fiber scope shaft 31. In this case, the protective cover 35 is used for covering the fiber scope shaft 1 including the lens 32, and the endoscope concerned in the present invention includes the fiber scope shaft 31 in the state covered with the protective cover 35.

The fiber scope shaft 31 is pliable and is constructed of a bundle of fibers consisting of a plural number of light guides (not shown) for irradiating light on the gastric wall SW and an image guide (not shown) for transmitting the reflected light via the lens 32. The joint portion 33 is connected to wiring 33a connecting the light guide to a light source device (not shown) and wiring 33b connecting the image guide to an image display device (not shown).

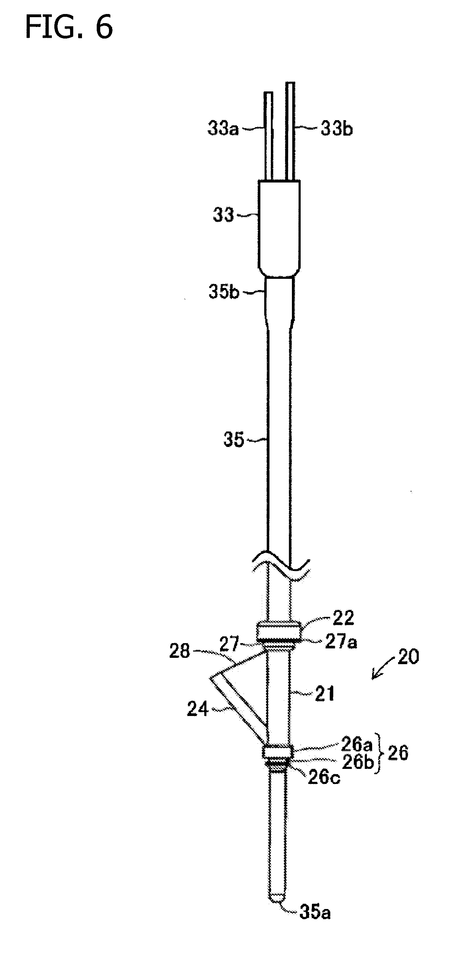

Further, the protective cover 35 is closed with a light transmittable window portion 35a at its tip portion and is made of a tube wherein the base end portion 35b at the opening side has a slightly larger diameter than that of the remaining portion, exhibiting pliability. This protective cover 35 is formed to have such a thickness capable of covering the lens 32 and the fiber scope shaft 31 and is attached to the fiber scope shaft 31 by inserting the tip slender portion 33c of the joint portion 33 into the base end portion 35b. In this case, the protective cover 35 is stopped from passing through the fiber scope shaft 31 using a member such as a clamp, tightening tool or stopper tool. In such a state, it is constructed such that the lens 32 becomes in contact with the interior surface of the window portion 35a. The fiber scope 31, as shown in Fig. 6, is inserted through the insertion through-hole 25 of the insertion assisting tool 20 in such a state that the fiber scope shaft 31 is covered with the protective cover 35. In this case, the space between the outer circumferential surface of the protective cover 35 and the inner circumferential surface of the cylindrical body 21 is sealed liquid-tightly or air-tightly by means of the sealing member 23.

Next, a method of checking the insertion position of the gastric fistula catheter 10 using the insertion assisting tool 20 having the aforementioned construction will be explained with reference to Figures 7 through 9. Fig. 7 shows a status of the gastric fistula catheter 10 inserted in the fistula set between patient's abdominal wall AW and the gastric wall SW, and the gastric fistula catheter 10 has been inserted into the fistula using a specific installation instrument. Explanation of the structure of the installation instrument and an insertion method are omitted. In the state shown in Fig. 7, the plug portion 16 of the gastric fistula catheter 10 is removed from the insertion hole 14 so that the upper end of the insertion 14 is open.

Further, the fiber scope 30 that has been inserted into the insertion assisting tool 20 along with the protective cover 35 is positioned above the gastric fistula catheter 10. The fiber scope 30 that in such a state along with the insertion assisting tool 20 is lowered as indicated by an arrow such that the fiber scope 30 projected from the lower end of the insertion assisting tool 20 along with the protective cover 35 is inserted into the supply path from the insertion hole 14 of the gastric fistula catheter 10. In this case, both side surfaces of the insertion inlet portion 11a in the gastric fistula catheter 10 are held by one hand, while the other hand holds the cylindrical body 21 of the insertion assisting tool 20 to press the insertion assisting tool 20 against the gastric fistula catheter 10.

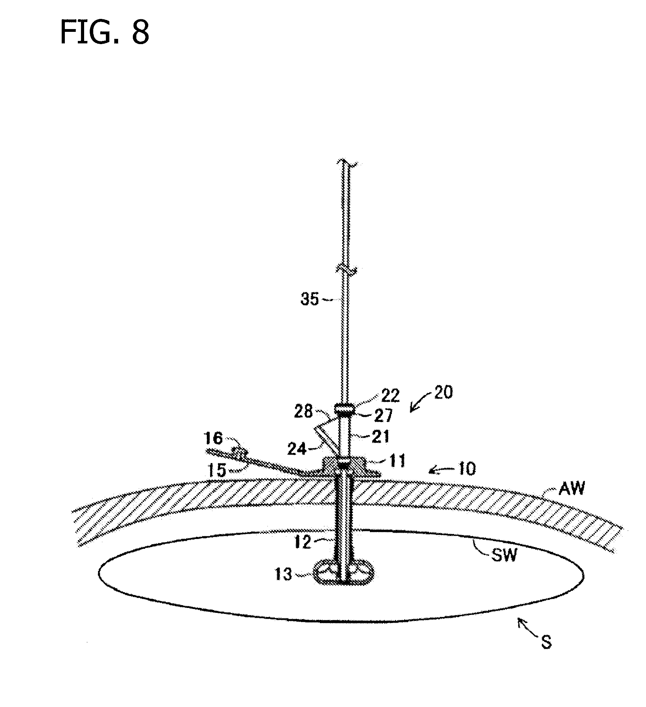

Accordingly the insertion assisting tool 20 is engaged with the gastric fistula catheter 10 as shown in Fig. 8. In this case, the engagement is made by engaging the ring-shaped projection portion 26c of the insertion assisting tool 20 with the engagement groove portion of the gastric fistula catheter 10, forming a through hole air-tightly or liquid tightly in the space between the insertion assisting tool 20 and the gastric fistula catheter 10. Further, the lower end portion of the engaged portion 26b expands the slit of the valve body 14a formed in the insertion hole 14 such that the outer circumferential surface of the engaged portion 26b and the circumferential rim portion can be sealed tightly. In this case, the screw status of the valve press member 22 against the cylindrical body 21 is slightly loosened such that the protective cover 35 along with the fiber scope 30 can be moved relative to the insertion assisting tool 20 while leakage in the space between the insertion assisting tool 20 and the protective cover 35 is prevented.

Subsequently, the fiber scope 30 along with the protective cover 35 is further inserted into the lower side of the gastric fistula catheter 10 and the lower side portion of the fiber scope 30 and the protective cover 35 is projected downward from the insertion through-hole 18 formed at the lower end portion of the gastric fistula catheter 10. In this case, the protective cover 35 is moved while being in close contact with the sealing member 23 of the insertion assisting tool 20. In addition, both fiber scope 30 and the protective cover 35 can be passed through the inside of the gastric fistula catheter 10 from the insertion assisting tool 20 after connecting the insertion assisting tool 20 to the gastric fistula catheter 10.

Next, the valve press member 22 is rotated to tighten it against the cylindrical body 21 in order to fix the fiber scope 30 and the protective cover 35 against the insertion assisting tool 20. In addition, air is supplied from the air supply device into the branching tube 24 and the air is blown from the insertion assisting tool 20 into the inside of the stomach S via the tube form 12 of the gastric fistula catheter 10. Accordingly, the stomach is swollen as shown in Fig. 9. In this state, the light generated from the light source is irradiated towards the gastric wall SW as shown in Fig. 9 passing through the wiring 33a and the light guide of fiber scope shaft 31.

Further, the reflected light from the gastric wall SW as irradiated by the light guide is converged through the lens 32 to be transmitted to the image display device via the image guide of the fiber scope shaft 31 and the wiring 33b. The reflected light transmitted to the image display device forms an enlarged image to be displayed on the image display. For this reason, whether or not the intra-gastric fixing portion 13 of the gastric fistula catheter 10 is set up properly in the stomach S can be checked by the image displayed on the image display.

If it is confirmed that the gastric fistula catheter 10 is inserted properly, both the fiber scope 30 and the protective cover 35 are removed from the gastric fistula catheter 10 along with the insertion assisting tool 20, while the insertion assisting tool 20 and the protective cover 35 are removed from the fiber scope 30 at the same time. This operation is carried out as follows. First, the valve press member 22 is turned to be loosened relative to the cylindrical body 21 such that the protective cover 35 and the fiber scope 30 can be moved from the insertion assisting tool 20. Next, both the fiber scope 30 and the protective cover 35 are listed upwards and when the status shown in Fig. 8 is attained, the engagement between the ring-shape projection portion 26c of the insertion assisting tool 20 and the engaged groove portion of the gastric fistula catheter 10 is released.

Further, both the fiber scope 30 and the protective cover 35 along with the insertion assisting tool 20 are pulled upwards to be withdrawn from the gastric fistula catheter 10. Furthermore, the insertion assisting tool 20 is removed from the fiber scope 30 and then the fiber scope 30 is withdrawn from the protective cover 35. Subsequently, the protective cover 35 is discarded and the fiber scope 30 is recycled for the next chance to be used. In this case, the lens 32 and the fiber scope shaft 31 are not stained since they are not directly in contact with the liquids and residues in the patient's body and the stomach S. Therefore, it is almost unnecessary to perform treatments such as washing and sterilization.

In addition, when recycling the fiber scope 30 for reuse, the fiber scope shaft 31 is covered with a new protective cover 35. In this case, in the aforementioned operation, the engagement of the ring-shape projection portion 26c of the insertion assisting tool 20 and the engaged groove portion of the gastric fistula catheter 10 is released and then the protective cover 35 and the fiber scope 30 are withdrawn along with the insertion assisting tool 20 from the gastric fistula catheter 10, but the protective cover 35 is withdrawn first from the insertion assisting tool 20 and then the engagement of the ring-shape projection portion 26c of the insertion assisting tool 20 and the engaged groove portion of the gastric fistula catheter 10 can be released next.

Also, for example, if liquid nutrients are supplied to the patient's stomach S via the gastric fistula catheter 10 inserted into the patient's body, a connector portion of the tube extended from the container containing the nutrients is connected to the insertion hole 14 of the gastric fistula catheter 10. The nutrients are supplied to the patient via the tube and the gastric fistula catheter 10 in this state. In this case, the nutrients coming from the tube-form portion 12 pass through the spaces among the respective band-shaped curved portion 13a from the interior of the intra-gastric fixing portion 13 to enter the interior of the stomach S. In addition, after supplying the nutrients, the tube of the nutrient container is removed from the insertion hole 14 of the gastric fistula catheter 10 and the insertion hole 14 is closed with the plug portion 16. Further, due to the uses for the specified period, when it is necessary to replace the gastric fistula catheter 10, it is replaced with a new gastric fistula catheter 10. In this case, the insertion position of the gastric fistula catheter 10 is checked again using the aforementioned insertion assisting tool 20 and the fiber scope 30.

According to the insertion assisting tool 20 concerned in this embodiment, the sealing member 23 is pressed against the fiber scope shaft 31 via the protective cover 35 according to the pressed pressure received from the valve press member 22 so that the space between the cylindrical body 21 and the protective cover 35 can be sealed securely.
Accordingly, leakage of air and blood and the like from the interior of the stomach S to the body can be prevented securely. If a greater pressure against the protective cover 35 presses the sealing member 23, the fiber scope shaft 31 can be fixed along with the protective cover 35.

In this case, clamping of the valve press member 22 against the cylindrical body 21 is adjusted by screwing between the screw thread 22b and the screw thread 27b so that the operation is easy. Further, according to this operation, not only the leakage of air and blood and the like from the interior of the stomach to the outside of the body can be prevented securely using the insertion assisting tool 20, but also the fiber scope 30 can be fixed at an appropriate position. Therefore, the insertion position of the gastric fistula catheter 10 can be checked accurately. In addition, a branched tube 24 is installed in the insertion assisting tool 20 for carrying air into the inside of the cylindrical body 21 so that air can be carried into the inside of the stomach S via the insertion assisting tool 20 even in the case of a fiber scope 30 without having an air carriage lumen for carrying air into the inside of the stomach S.

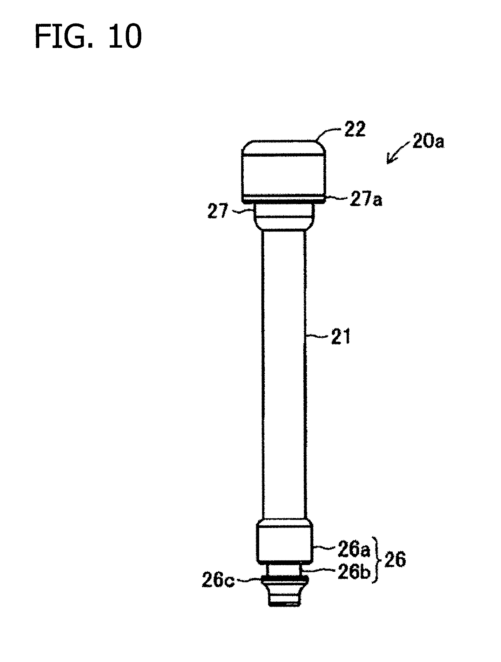

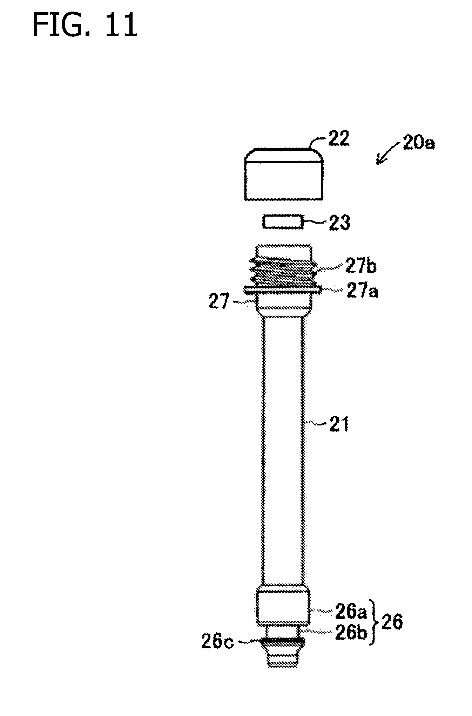

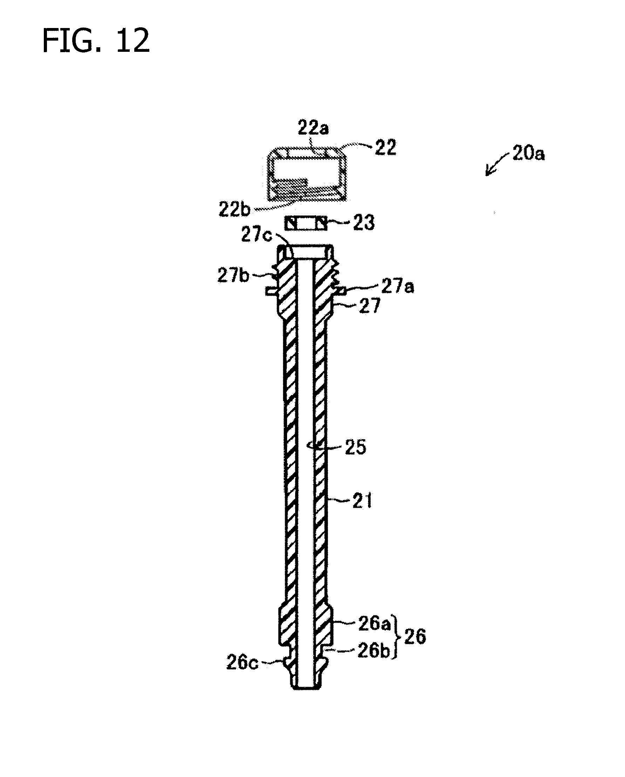

In the second embodiment of the present invention, Figures 10 through 12 show an insertion assisting tool 20a concerned. Neither the branched tube 24 or the reinforcement grip portion 28 are installed in this insertion assisting tool 20a, but the constitution of other portions is identical to that of the insertion assisting tool 20 concerned in the aforementioned first embodiment. Therefore, the same symbols are used for the same portions and their explanation is omitted. This insertion assisting tool 20a is used in the case when using another endoscope having an air carriage lumen instead of the fiber scope. Therefore, air is supplied into the stomach S via the air carriage lumen of the fiber scope.
The effects of actions other than those of the insertion assisting tool 20a are the same as those of the insertion assisting tool 20 concerned in the aforementioned first embodiment.

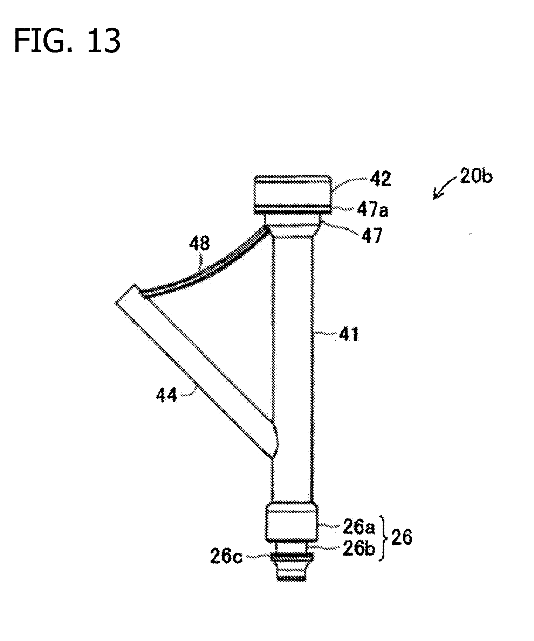

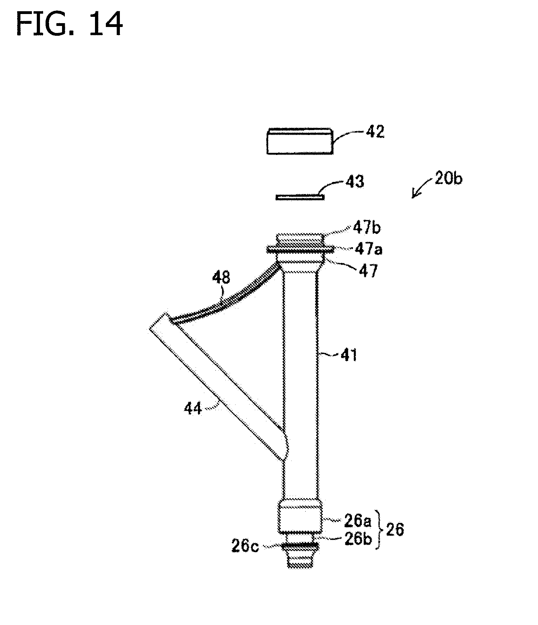

In the third embodiment of the present invention, Figures 13 and 14 show an insertion assisting tool 20b concerned. In this insertion assisting tool 20b, its upper side portion is formed in a cylindrical shape that is shorter in length in the axial direction than the stopper portion 47a in the insertion inlet portion 47 formed at the upper end portion of the cylindrical body 41, and a ring-shaped engagement projection portion 47b is formed on its outer circumferential surface instead of a screw. Further, a storage concave portion is not formed on the upper end internal circumferential surface of the insertion inlet portion 47. In addition, the valve press part 42 is constructed in a cap shape having a hole portion that is shorter in length in the axial direction than the valve press member 22, and a ring-shaped engaged concave portion (not shown) that can be engaged with the engaged projection portion 47b is formed on the internal circumferential surface.

A branched tube 44 is formed in a cylindrical shape extending upward diagonally in an inclined state with an angle of approximately 45 degrees relative to the cylindrical body 41 from the lower side portion in the axial direction of the cylindrical body 41. A nearly triangular thin plate reinforcement grip portion 48 is formed in the space between the cylindrical body 41 and the branched tube 44 in order to hold the insertion assisting tool 20b easily with hands. The upper end rim portion of the reinforcement grip portion 48 is formed in a concave curved rim portion with an indentation in the center. Further, a sealing member 43 is formed in a ring-shape with a less thickness and the outer diameter of the sealing member 43 is almost equal to the outer diameter of the upper end surface of the insertion inlet portion 47.

The constitution of other portions in this insertion assisting tool 20b is identical to that of the insertion assisting tool 20 concerned in the aforementioned first embodiment. Therefore, the same symbols are used for the same portions and their explanation is omitted. The insertion assisting tool 20b is assembled by placing the sealing member 23 to be fitted along the upper end surface of the cylindrical body 41, or in the state along the sealing portion by placing into the interior of the valve press member 42 by engaging the engaged concave portion of the valve press member 42 with the engaged projection portion 47b of the cylindrical body 41. The effects of actions other than those of the insertion assisting tool 20b are the same as those of the insertion assisting tool 20 concerned in the aforementioned first embodiment.

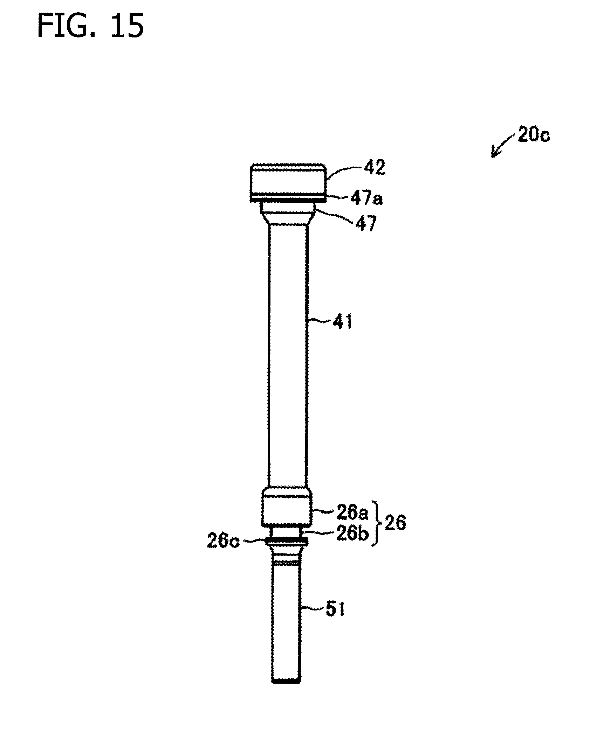

In the fourth embodiment of the present invention, Figure 15 shows an insertion assisting tool 20c concerned. This insertion assisting tool 20c contains an extension portion 51 extending downward the lower portion of the joint portion 26 in the insertion assisting tool 20b in the aforementioned second embodiment, and the branched tube 44 and the reinforcement grip portion 48 are omitted in this constitution. The constitution of other portions besides the insertion assisting tool 20c is identical to that of the insertion assisting tool 20b. Therefore, the same symbols are used for the same portions and their explanation is omitted. This insertion assisting tool 20c is used in the case when using a gastric fistula catheter wherein the valve body 14a is set on the internal circumferential surface of the tube-form portion 12 instead of on the internal circumferential surface of the insertion hole 14 of the outer fixing portion 11. In this case, when installing the insertion assisting tool 20c in the gastric fistula catheter, the extension portion 51 presses the valve body to open the slit. The effects of actions other than those of the insertion assisting tool 20c are the same as those of the insertion assisting tool 20b concerned in the aforementioned third embodiment.

Further, the insertion assisting tool for an endoscope concerned in the present invention is not limited by the aforementioned embodiments and a variety of changes can be made within the technological scope of the present invention. For example, a protective cover 35 covers the fiber scope shaft 31 of the fiber scope 30 in the aforementioned embodiments, but a fiber scope shaft 31 can be inserted directly into the stomach S without using this protective cover 35. In addition, air is supplied into the stomach S respectively from the branched tube 24 in the insertion assisting tool 20 in the aforementioned first embodiment and from the branched tube 44 in the insertion assisting tool 20b in the third embodiment, but indigo carmine for staining the gastric wall SW and the gastrointestinal inner wall or liquids for accelerating or inhibiting vermiculation of the stomach S can be supplied from the branched tubes 24 and 44 into the stomach S.

Furthermore, the fiber scope 30 is used to check the insertion position of the gastric fistula catheter 10 in the aforementioned embodiments, but this fiber scope 30 can be used to check the status of the gastric wall SW. In addition, various devices such as image display devices and light source devices and the like can be altered to other devices having similar functions. Moreover, a wire is attached to the tip side portion of the protective cover 35 and the tip side portion of the protective cover 35 can be bent along with the fiber scope shaft 31 by pulling this wire, or a fiber scope 30 having a mechanism for bending the tip portion can be used. According to this, the tip portion of the fiber scope 30 can be directed in a variety of directions.

Further, in the aforementioned embodiments, the valve press member 22 is installed detachably by screwing the screw threads 22b and 27b, or the valve press member 42 is installed detachably by engaging the engaged concave portion with the engaged projection portion 47b, but they can be fixed by bonding. Moreover, the ring-shaped member does not only limit a sealing member, but also a cylindrical member can be used.

The insertion assisting tool for an endoscope concerned in the present invention with the aforementioned constitution in one embodiment comprises a cylindrical body that can be engaged air tightly or liquid tightly with the outer fixing portion of the gastric fistula catheter wherein an endoscope can be passed through and a sealing member attached to the cylindrical body. For this reason, an endoscope can be passed through the inside of the cylindrical body engaged air tightly or liquid tightly with the outer fixing portion of the gastric fistula catheter. In this case, since the space between the cylindrical body and the endoscope is sealed with a sealing member, the space between the gastric fistula catheter and the insertion assisting tool for the endoscope and the space between the endoscope and the insertion assisting tool for the endoscope are sealed respectively such that air leakage from the interior of the stomach to the outside of the body can be prevented.

Further, the engagement between the outer fixing portion of the gastric fistula catheter and the cylindrical body can be made by engaging a projected portion with a concave portion or by screwing together. Furthermore, the portion where the cylindrical body of the endoscope passes through may be the portion that is at least inserted into patient's body. As a sealing member, a ring-shaped member or a cylinder-shaped member can be used.

Further, another structural characteristic of one embodiment of the insertion assisting tool for the endoscope of the present invention is that a cap-shaped valve press member having a perforated portion on the sealing portion passing the endoscope is-attached at the opposite end of the portion engaged with the outer fixing portion in the cylindrical body, while a sealing member is installed between the end portion of the cylindrical body and the valve press member in order to seal the space between the cylindrical body and the endoscope.

In this case, the valve press member can be attached at variable locations relative to the cylindrical body by fixing it on the cylindrical body by bonding, or by engaging a projected portion with a concave portion, or by screwing both screws. If the valve press member is attached at variable locations on the cylindrical body, the sealing member is pressed against the endoscope according to the pressure applied from the valve press member so that sealing of the space between the cylindrical body and the endoscope can be secured. It is also possible to further increase the difficulty for the endoscope to shift from the insertion assisting tool for the endoscope by increasing the frictional resistance of the space between the sealing member and the endoscope. Furthermore, attachment of a sealing member becomes easier by installing a sealing member in the space between the end of the cylindrical body and the valve press member.

Further, the other structural characteristic of one embodiment of the insertion assisting tool for the endoscope of the present invention is that the valve press member is installed at an adjustable position in an axial direction of the cylindrical body such that the position of the valve press member is adjusted relative to the cylindrical body wherein the endoscope inserted into the cylindrical body can be fixed relative to the cylindrical body. According to this structure, not only does the insertion assisting tool for an endoscope prevent air leakage from the interior of the stomach to the outside of the body, but also the endoscope can be fixed at an appropriate position. Therefore, the status of the interior of the stomach and the insertion position of the gastric fistula catheter can be checked accurately.

Further, the other structural characteristic of one embodiment of the insertion assisting tool for the endoscope of the present invention is that a branching tube for sending fluid into the inside of the cylindrical body is installed on the outer circumferential surface of the cylindrical body. According to this structure, even though the endoscope does not have a gas carriage lumen for carrying air into the interior of the stomach, air can be carried into the interior of the stomach via the insertion assisting tool for an endoscope. Furthermore, indigo carmine for staining the gastric wall and liquid agents for accelerating or inhibiting vermiculation of the stomach can be supplied from this branching tube into the interior of the stomach.

Further, the other structural characteristic of one embodiment of the insertion assisting tool for the endoscope of the present invention is that a valve body is installed in the insertion hole of the outer fixing portion in order to open/close the insertion hole such that the cylindrical body presses to open the valve body when the cylindrical body is engaged with the outer fixing portion. According to this structure, an engagement between the outer fixing portion of the gastric fistula catheter and the cylindrical body of the insertion assisting tool for an endoscope can be further stabilized and the sealing between the outer fixing portion and the cylindrical body can be further secured. Furthermore, when passing the endoscope through the interior of the gastric fistula catheter, this structure can prevent the insertion of the endoscope from becoming difficult due to the contact of the peripheral surface of the endoscope with the valve body.

Further, the other structural characteristic of one embodiment of the insertion assisting tool for the endoscope of the present invention is that a valve body is installed in the internal cavity of the tube-form portion in order to open/close the internal cavity such that when passing the cylindrical body through the tube-form portion, the cylindrical body presses and opens the valve body. According to this structure, an engagement between the outer fixing portion of the gastric fistula catheter and the cylindrical body of the insertion assisting tool for an endoscope can be further stabilized and the sealing between the outer fixing portion and the cylindrical body can be further secured. Furthermore, when passing the endoscope through the interior of the gastric fistula catheter, this structure can prevent the insertion of the endoscope from becoming difficult due to the contact of the peripheral surface of the endoscope with the valve body.

## Claims

1. An insertion assisting tool for an endoscope comprising a tube-form portion having an internal cavity and an outer fixing portion forming an insertion hole passing through the cavity of the said tube-form portion inside connected to a base end of the said tube-form portion, which can be used when inserting an endoscope into the gastric fistula catheter that has been inserted in the said fistula while said tube-form portion is positioned in the fistula formed in the space between the patient's skin surface and the inner surface of the gastric wall, **characterized in that** it comprises a cylindrical body that can be engaged air-tightly or liquid-tightly with said outer fixing portion and that can pass said endoscope inside, and a sealing member attached to said cylindrical body for sealing the space between said cylindrical body and said endoscope.

2. The insertion assisting tool for an endoscope as described in Claim 1 wherein a cap-form valve press member forming a hole portion that can pass said endoscope in the sealing portion can be installed at the edge portion at an opposite side of the portion engaged with said outer fixing portion in said cylindrical body, and said sealing member can be set in the space between said edge portion of said cylindrical body and said valve press member such that the space between said cylindrical body and said endoscope can be sealed.

3. The insertion assisting tool for an endoscope as described in Claim 2 wherein said valve press member can adjust the position in an axial direction of said cylindrical body and said endoscope inserted into said cylindrical body can be fixed on said cylindrical body by adjusting the position of said valve press member relative to said cylindrical body.

4. The insertion assisting tool for an endoscope as described in Claim 1 wherein a branched tube for carrying fluids into the inside of said cylindrical body can be installed on the outer circumferential surface of said cylindrical body.

5. The insertion assisting tool for an endoscope as described in one of Claim 1 wherein a valve body for opening and closing said insertion hole can be set in the insertion hole of said outer fixing portion and when said cylindrical body is engaged with said outer fixing portion, said cylindrical body presses to open said valve body.

6. The insertion assisting tool for an endoscope as described in Claim 1 wherein a valve body for opening and closing said internal cavity can be set in the internal cavity of said tube-form portion and when said cylindrical body passes through said tube-form portion, said cylindrical body presses to open said valve body.
